Europäisches Patentamt

European Patent Office

Office européen des brevets

(19)

(11) Numéro de publication: **0 574 398 B1**

(12) **FASCICULE DE BREVET EUROPEEN**

(45) Date de publication du fascicule du brevet: **23.08.95**  (51) Int. Cl.6: **A61K 39/395, A61K 9/12**

(21) Numéro de dépôt: **92901306.8**

(22) Date de dépôt: **28.11.91**

(86) Numéro de dépôt internationale :
**PCT/FR91/00946**

(87) Numéro de publication internationale :
**WO 92/09306 (11.06.92 92/13)**

(54) **UTILISATION D'IMMUNOGLOBULINES ADMINISTREES LOCALEMENT DANS LE TRAITEMENT DES INFECTIONS EPITHELIALES.**

(30) Priorité: **28.11.90 FR 9014881**

(43) Date de publication de la demande:
**22.12.93 Bulletin 93/51**

(45) Mention de la délivrance du brevet:
**23.08.95 Bulletin 95/34**

(84) Etats contractants désignés:
**AT BE CH DE FR GB IT LI SE**

(56) Documents cités:
**WO-A-89/00428**
**US-A- 4 800 078**
**US-A- 4 994 269**

**BIOLOGICAL ABSTRACTS, V 86, 1988; D. BESSEN et al., AN 69797/**

(73) Titulaire: **FONDATION NATIONALE DE TRANS-FUSION SANGUINE**
**6, rue Alexandre Cabanel**
**F-75739 Paris Cédex 15 (FR)**

(72) Inventeur: **ALONSO, Jean-Michel**
**51, rue de Cornouailles**
**F-78180 Montigny-le-Brétonneux (FR)**
Inventeur: **COURTEILLE, Frédéric**
**9, rue du Docteur-Hénouille**
**F-94230 Cachan (FR)**
Inventeur: **BINDER, Patrice**
**22, rue Lavoisier**
**F-91710 Vert-le-Petit (FR)**
Inventeur: **RAMISSE, Françoise**
**7, route de la Ferté-Alais**
**F-91590 D'Huisson-Longueville (FR)**

(74) Mandataire: **Warcoin, Jacques et al**
**Cabinet Régimbeau,**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne l'utilisation d'Immunoglobulines administrées localement dans le traitement des infections épithéliales. Les infections épithéliales sont causées par des agents bactériens ou viraux, pathogènes ou opportunistes, capables de proliférer in situ, durant les étapes précoces de l'infection. Cette phase de colonisation des tissus-cibles peut se compliquer ou non de lésions proximales ou de dissémination. Cette phase de l'infection est en outre, la plus dangereuse pour la transmission d'hôte infecté à hôte réceptif, et détermine ainsi le déclenchement d'épidémies.

L'induction du mécanisme déclenchant de ces infections est le fait d'interactions moléculaires, entre d'une part, des factures d'adhésion de l'agent pathogène et, d'autre part, des recepteurs exprimés à la surface des cellules de l'hôte.

Tout élément inhibant cette interaction primaire immunise sur le déclenchement de l'infection.

Les infections épithéliales se manifestent anatomiquement pour tous les tissus de revêtement de l'organisme. Les épithéliums muqueux sont des sites d'infections plus ou moins spécifiques selon leur fonction et selon la flore saprophyte.

Chez l'hôte immuno-compétent, les défensess contre ces infections sont assurées par des immunoglobulines à fonction anticorps, secrétées in situ (IgAs, IgG, IgM).

Chez l'hôte immunodéficient ou immuno-immature (cas des jeunes enfants),, ces effecteurs sont absents ou non fonctionnels.

Sur le plan épidémiologique, les infections respiratoires de l'enfant constituent un bon exemple de cette condition. Leur incidence décroissante avec l'âge est en corrélation avec une maturation du système immunitaire et l'acquisition d'une immunité naturelle spécifique.

Le brevet US- 4 800 078 a proposé le traitement des infections respiratoires à VRS par administration de gammaglobulines spécifiques anti VRS.

Dans le Biological Abstract, V. 86, 1988, n° 69797, il est indiqué que seules les IgA sécrétoires ont une activité locale, alors que les immunoglobulines sériques n'induisent pas de protection.

Chez l'adulte sain, les formations lymphoïdes associées aux épithéliums secrètent des anticorps locaux neutralisant une variété d'agent infectieux contre lesquels l'organisme est immunisé.

Ces spécificités-anticorps locales sont également présentes dans le plasma des adultes cliniquement sains. Ceux-ci constituent la population sélectionnée des donneurs de plasma.

L'objet de la présente invention est notamment l'utilisation des anticorps purifiés du plasma de donneurs adultes sains pour prévenir et traiter par voie locale les infections épithéliales et notamment les principales infections bactériennes respiratoires et ORL de l'enfant. Celles-ci sont majoritairement provoquées par Staphylococcus aureus, Streptococcus pyogenes, Streptococcus pneumoniae et Hemophilus influenzae.

Selon les sources mondiales (WHO memorandum, Bull World, Hlth Org. 1984, 62:47), les infections respiratoires aigues constituent une cause majeure de mortalité infantile. L'incidence globale des infections respiratoires contribue, selon les pays, à 20% à 40% des motifs de consultation en pédiatrie et à 12-35% des motifs d'hospitalisation.

Le caractère multifactoriel de ces infections rend difficilement concevables des vaccinations spécifiques.

Les traitements anti-microbiens sont largement appliqués (béta-lactamines et macrolides, par exemple) mais ils posent de plus en plus le problème de la sélection/induction et donc de l'émergence de variants résistants. Des immunostimulants à base d'extraits d'antigènes bactériens se révèlent efficaces. Mais le besoin d'une immunothérapie supplétive qui permette de conférer une protection immédiate, sans solliciter l'arsenal des cellules immunocompétentes dans son ensemble et ce quelle que soit la compétence ou maturité immunitaire du patient, reste non couvert.

Les Ig polyvalentes, purifiées du plasma de donneurs adultes sains, sont largement et efficacement employées par voie parentérale (IM ouIV) contre les déficits immunitaires.

Cependant, l'administration parentérale ne semble pas garantir le passage des anticorps seriques vers le compartiment épithélial (Abraham, SN and Beachey, EH, in Advances in Host Defense Mechanisms, 1985, 4:63). C'est pourquoi, la présente invention concerne l'application locale de tels anticorps.

Plus particulièrement la présente invention concerne l'utilisation d'au moins un effecteur parmi les immunoglobulines polyvalentes leur fragment Fab'2 ou Fab et les constructions incorporant ces éléments pour la réalisation de compositions pharmaceutiques applicables par voie topique pour le traitement des infections épithéliales.

De façon générale, on préférera utiliser des immunoglobulines polyclonales ou monoclonales ou un fragment de ces immunoglobulines notamment celles d'origine plasmatique.

Les immunoglobulines utilisées sont plus particulièrement des immunoglobulines polyvalentes ou leurs fragments, notamment les produits d'origine plasmatique humaine. Ces produits existent déjà pour l'injection, ils sont donc directement utilisables pour des administrations topiques notamment au niveau des voies respiratoires. La forme d'administration préférée est l'administration par voie nasale, mais on peut envisager des aérosols oro-pharyngés. La mise en forme galénique des formes topiques internes est connue aussi bien pour les aérosols que pour les formes goutte par exemple.

Ainsi on pourra adjoindre aux effecteurs, des composants améliorant leur contact avec les épithéliums à traiter, notamment des produits de type tensio-actifs.

Les compositions selon la présente invention peuvent être utilisées aussi bien à titre préventif qu'à titre curatif et plus particulièrement pour le traitement des infections bactériennes respiratoires et ORL de l'enfant, et de façon générale chez les sujets présentant des déficiences immunitaires.

Les immuglobulines polyvalentes sont particulièrement utiles dans le traitement des injections bactériennes à Staphylocoque, et à Streptocoque, comme cela sera démontré dans les exemples.

Les exemples suivants ont notamment pour objet de mettre en évidence d'autres caractéristiques et avantages de l'invention notamment par administration locale d'Ig polyvalentes à titre préventif ou curatif pour neutraliser l'infection pulmonaire par Staphylococcus aureaus, Streptococcus pneumoniae, Streptococcus pyogenes et Hemophilus influenzae.

Sur les figures ci-annexées:

- la figure 1 schématise la cinétique d'élimination de la souche de S. aureus Cowan III, après instillations nasales chez des souris normales et traitées au CY.
- La figure 2 schématise les effets comparés par rapport à un groupe témoins de l'administration des GVP par voie I.V. et I.N.
- la figure 3 schématise les essais GVP H + 3, souche 184-15.
- la figure 4 schématise les essais GVP H + 18, souche Cowan III.
- la figure 5 schématise les essais GVP H-1, souche Cowan III.
- la figure 6 schématise les essais (GVP-PA), souche Cowan III.

## MATERIELS ET METHODES

### Animaux

On utilise des souris Balb/c femelles agées de 4 semaines, provenant de l'élevage du CERJ (Le Genest St Isle 53940). Elles sont groupées par lots de 3 ou 6, dans des conditions stériles : litière composée de copeaux stériles, eau et aliments stériles, cages recouvertes de capes filtrantes (fournisseur UAR : cages Techniplast type 2C code 1264C0-000 : capes filtrantes code 1264C0-401 ; granulés réf R0-340 ; copeaux réf 40). L'animalerie, maintenue en dépression par rapport à l'air ambiant, est entreposée dans un laboratoire type P3. Pour les infections expérimentales par instillation nasale, elles sont légèrement anesthésiées à l'éther ; elles sont sacrifiées par injection intrapéritonéale de 200 ul chacune de pentobarbital sodique (Sanofi) au moment de prélever les poumons.

Souches bactériennes

- Staphylococcus aureus 184-15 isolée d'un malade atteint de pneumonie,
- Staphylococcus aureus CIP 82/03, létale pour la souris par voie intrapéritonéale,
- Staphylococcus aureux Cowan III CCM 885 CIP 65/8, ATCC 12600.
- Streptococcus pyogenes groupe A CIP 56/48, ATCC 12203,
- Streptococcus pyogenes groupe A CIP 56/41, ATCC 12344,
- Streptococcus pneumoniae référence Pn 40, laboratoire des Streptocoques, Institut Pasteur T. Horaud,
- Haemophilus influenzae CIP 54/81, ATCC 9006.

Conditions de préparation de l'inoculum infectieux

### Les souches de staphylocoques

Ces bactéries sont cultivées en bouillon trypticase soja (DIFCO) ; une culture de densité suffisante est obtenue après une nuit à 37°C, sous agitation régulière de 100 tours/min. La souche 82/03 a été "passée sur souris" après injection intrapéritoniale, puis ré-isolement sur gélose au sang, à partir de prélèvements de poumons ou de rate, afin d'augmenter sa virulence. Elle a été à nouveau éprouvée dans le cas d'inoculation par voie nasale, puis par aérosol.

**Les souches de streptocoques**

Afin d'obtenir une suspension bactérienne concentrée, 5 géloses enrichies avec 10% de sang stérile de cheval (Diagnostics Pasteur) sont ensemencées et incubées à 37°C pendant une nuit. Le lendemain les boîtes sont raclées avec une tige cotonnée stérile, et les bactéries ainsi prélevées sont mises en suspension dans 4 ml de tampon PBS stérile. La composition de ce soluté aqueux est la suivante : NaCl : 0,15 M ; KCl : $2,7 \times 10^{-3}$ M ; $KH_2PO_4$ : $1,4 \times 10^{-3}$ M ; $Na_2HPO4$ : $9 \times 10^{-3}$ M.

**La souche d'Haemophilus influenzae**

La suspension bactérienne de cette souche est obtenue de la même façon que précédemment, mais après culture de 48 heures sur gélose à l'extrait globulaire 10% (Diagnostics Pasteur).

Méthodes de mesure des cinétiques infectieuses

**Les prélèvements de poumons**

A différents temps après instillations, les poumons des souris (par groupes de 3 à 6) sont prélevés stérilement et broyés dans 1 ml de tampon PBS. Les prélèvements sont effectués 1 heure, 3 heures, 6 heures, 24 heures, 48 heures, 72 heures et jusqu'a élimination complète des bactéries, chez les animaux traités.

**Les numérations de bactéries**

L'inoculum et les broyats sont dilués de 10 en 10 dans du tampon PBS ; 100 ul des trois ou quatre dernières dilutions sont étalés sur milieu trypticase - soja. L'incubation se fait pendant 18 à 48 heures à 37° selon la souche: après quoi le comptage des colonies est effectué.

Calcul du nombre d'unités formant colonie (U.F.C.)

- Soit N le nombre de colonies sur la boîte,
- soit d la dilution,
- la concentration bactérienne C par ml (ou par poumon de souris) s'établit comme suit :

$$C = N \times 1/d \times 10$$

C'est exprimé en $\log_{10}$.

Pour tous les points de la cinétique, la moyenne et la déviation standard sont calculées. Les résultats obtenus sont représentés graphiquement : log 10 UFC/ml = f (temps).

**L'immunodépression par le cyclophosphamide**

Afin d'obtenir des cinétiques de prolifération bactérienne durables, nous avons injecté par voie intraveineuse 500ul d'une solution de cyclophosphamide (CY) à 200 mg/Kg (Aldrich). Le cyclophosphamide agit comme un immunodépresseur diminuant les leucoytes totaux avec une activité cytotoxique sur les cellules B et T.

Il empêche notamment les cellules B de régénérer à un rythme normal leurs immunoglobulines de surface. (In Immunologie fondamentale et appliquée, ROITT I.M., et coll. ed. 1985 MEDSI, Paris, pp 12.8). Pour contrôler l'efficacité de ce produit, des formules sanguines ont été effectuées tous les jours pendant 10 jours. Les bactéries sont administrées par instillation le 3ème jour après l'injection de cyclopliosphamide.

Traitement par Ig

Les immunoglobulines polyvalentes du CNTS (GVP) lot n° 505 ont été administrées soit par voie veineuse (IV) soit par voie intranasale (IN) aux doses de 1 et 10 ug par souris, sous un volume de 50 ul.

Pouvoir neutralisant des Ig in vitro

Le pouvoir antibactérien des GVP a été évalué, dans le cas de Staphylococcus aureus (souche Cowan III) dans une épreuve d'inhibition de croissance en milieu liquide pendant 18 heures. Dans ces conditions et en absence de complément, l'inhibition est supérieure à 90%.

RESULTATS DES TRAITEMENTS PAR GVP

Afin de conférer une haute significativité aux effets mesurés, les épreuves sont réalisées chez des souris immunodéprimées par injection de cyclophosphamide (CY) à la dose de 200 mg/Kg, trois jours avant l'infection.

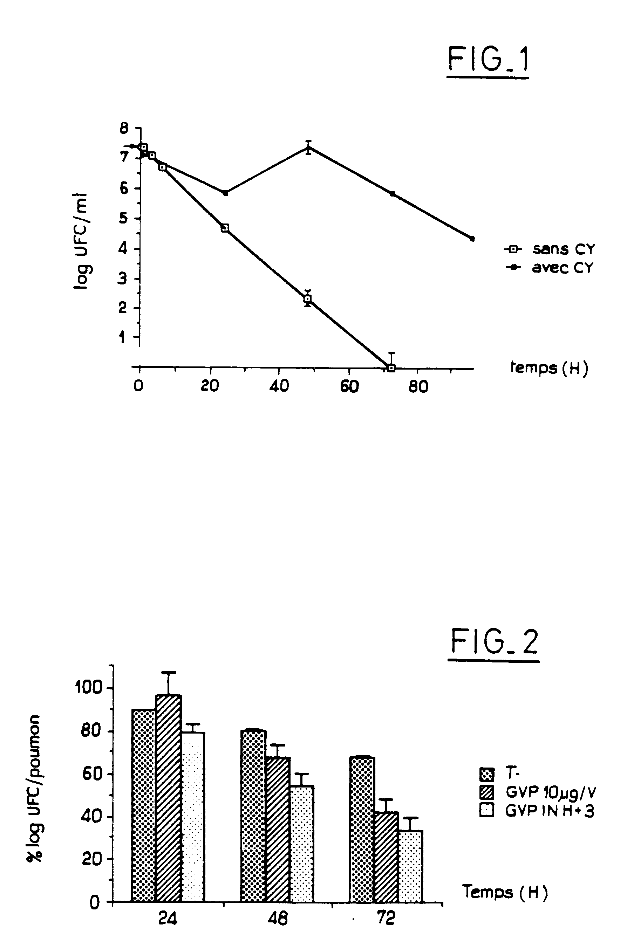

5 à $10 \times 10^7$ UFC de Staphylococcus aureus provoquent une infection prolongée au-delà de 96 h avec création d'une pneumonie massive dont les lésions sont macroscopiquement visibles dès 24 h. Une cinétique-type est présentée en figure 1.

L'installation i.n de 1 $\mu$g de GVP par souris, 3 h après induction (GVP IN H + 3)de l'infection est aussi efficace que l'administration iv de 10 $\mu$g (GVP 10 $\mu$g/iv) (figure 2).

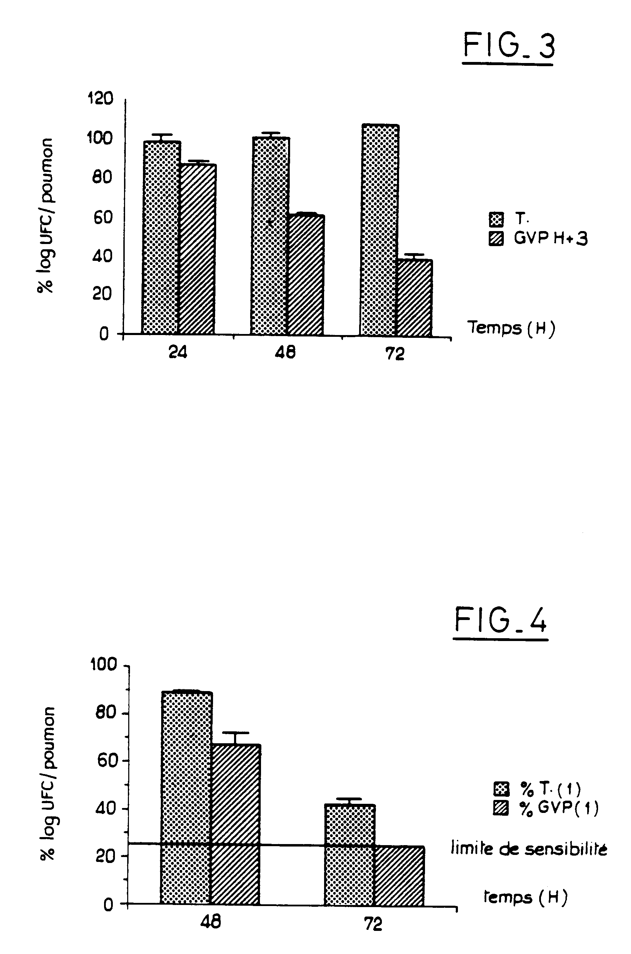

Comme le montre la figure 3, l'instillation de 1 ug de GVP est également efficace contre une souche de Staphylococcus aureus fraîchement isolée d'un malade, la souche 184-15.

Le traitement de la pneumonie à Staphylococcus aureus est efficace même à une phase avancée de l'infection. Comme le montre la figure 4, l'instillation in de 1 ug de GVP, 18 h après l'induction de l'infection, diminue de 10 fois le nombre de bactéries pulmonaires, à 48 à 72 h.

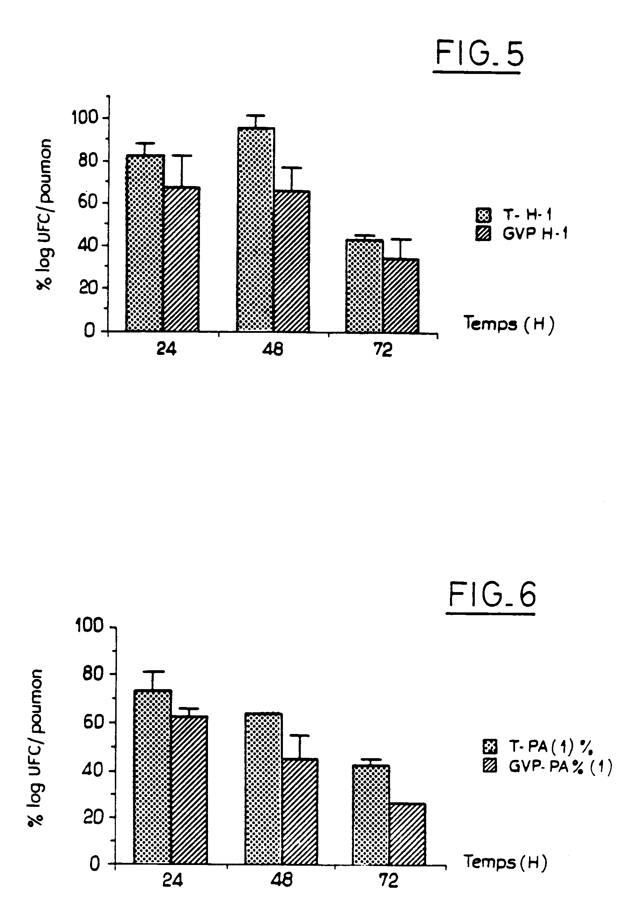

Il est possible de prévenir le développement d'une infection pulmonaire par S. aureus par instillation in de 1 ug de GVP. Comme le montre la figure 5, l'administration topique des GVP 1 h avant l'infection exerce un effet inhibiteur contre l'infection bactérienne et permet d'en accélérer la guérison.

Nous avons vérifié que l'activité neutralisante des GVP contre S. aureus n'est pas due a une interaction du fragment $F_c$ des Ig avec la protéine A. Comme le montre la figure 6, la saturation de la préparation de GVP par la protéine A de S. aureus (protéine A, Sigma) à la concentration finale de 1%, ne modifie pas son pouvoir immmunoprotecteur (les animaux ne recevant que la solution de protéine A).

L'effet anti-bactérien des GVP administrées par voie locale est également démontrable dans l'infection respiratoire des souris par S. pneumoniae et S. pyogenes (tableau 1), bien que dans ce dernier cas l'effet neutralisant soit apparent à 48 h au lieu de 24 h dans les exemples précédents.

En résumé, les préparations d'immunoglobulines plasmatiques humaines injectables, dont l'efficacité clinique est démontrée de longue date, peuvent être utilisées en tant qu'anticorps locaux pour traiter et prévenir des infections épithéliales chez le patient immunodéficient ou immuno-immature.

**TABLEAU I**

Effets neutralisants des GVP administrées par voie nasale, à la dose de 1μg (100μl/Kg) et à 3 h après l'injection intranasale de S. pneumoniae ou S. pyogenes.

No UFC / poumons ($log_{10}$)

| inoculum infectieux | 24 h | | 48 h | |
| --- | --- | --- | --- | --- |
| | Témoins | Traités | Témoins | Traités |
| S. pneumoniae Pn 40 | | | | |
| * expérience 1 inoculum 7.7 | 6.75+/-0.25 | 4.65+/-0.60 | ND* | ND* |
| * expérience 2 inoculum 5.0 | 5.20+/-0.40 | 4.40 | 5.0+/-1.1 | 4.38 |
| S. pyogenes 56.81 inoculum 8.3 | 4.90+/-0.40 | 5.50+/-0.60 | 5.50+/-0.40 | 4.0 |

*ND : donnée non mesurée

**Revendications**

1. Utilisation d'immunoglobulines polyvalentes d'origine plasmatique humaine, ou leurs fragments Fab'2 ou Fab, pour la réalisation de compositions pharmaceutiques préventives ou curatives applicables par voie topique pour le traitement des infections épithéliales.

2. Utilisation selon la revendication 1, caractérisée en ce que l'infection épithéliale traitée est une infection bactérienne respiratoire et ORL d'un sujet immunodéficient ou immuno-immature.

3. Utilisation selon l'une des revendications 1 et 2, caractérisée en ce que les immunoglobulines sont utilisées sous la forme administrable par voie nasale ou oro-pharyngée.

4. Utilisation selon l'une des revendications 1 à 3, caractérisée en ce que les immunoglobulines sont utilisées sous forme aérosol.

**Claims**

1. Use of polyvalent immunoglobulins of human plasma origin, or their 'F(ab')$_2$' or Fab fragments for the production of preventive or curative pharmaceutical compositions which can be applied topically for the treatment of epithelial infections.

2. Use according to Claim 1, characterized in that the epithelial infection treated is a respiratory and ENT bacterial infection of immunodeficient or immunologically immature subject.

3. Use according to one of Claims 1 and 2, characterized in that the immunoglobulins are used in the form which can be administered nasally or oropharyngeally.

4. Use according to one of Claims 1 to 3, characterized in that the immunoglobulins are used in the form of an aerosol.

**Patentansprüche**

1. Verwendung von polyvalenten Immunglobulinen aus dem Human-Plasma oder ihrer Fragmente Fab'2 oder Fab zur Herstellung von präventiven oder heilenden pharmazeutischen Zusammensetzungen, die auf topischem Wege appliziert werden können für die Behandlung von Epithel-Infektionen.

2. Verwendung nach Anspruch 1, dadurch gekennzeichnet, daß die behandelte Epithel-Infektion eine respiratorische Bakterien- und ORL-Infektion eines Patienten mit einem defekten oder nicht ausgereiften Immunsystem ist.

3. Verwendung nach einem der Ansprüche 1 und 2, dadurch gekennzeichnet, daß die Immunglobuline in einer auf nasalem oder oropharyngealem Wege verabreichbaren Form verwendet werden.

4. Verwendung nach einem der Ansprüche 1 bis 3, dadurch gekennzeichnet, daß die Immunglobuline in Form eines Aerosols verwendet werden.

FIG_1

FIG_2

## FIG_3

## FIG_4

## FIG.5

## FIG.6